# EUROPEAN PATENT APPLICATION

(11) **EP 2 471 464 A1**
(43) Date of publication of application: **04.07.2012**
(21) Application number: 11150062.5
(22) Date of filing: 04.01.2011
(51) Int. Cl.: A61B 17/02, A61B 19/00, A61B 17/00

(54) **Surgical or medical retractor device**

(71) Applicant: Universitätsklinikum Münster, 48149 Münster (DE)
(72) Inventor: Zscheile, Axel, 48341, Altenberge (DE); Boes, Josef, 48346, Ostbevern (DE); Rassoulian, Darius Dr. med., 40239, Düsseldorf (DE)
(74) Representative: Isarpatent

(57) **Abstract**

The invention relates to a surgical retractor device comprising: a frame, a shaft element coupled to the frame, wherein the shaft element is provided with an adjusting device which is movably arranged along the shaft element to be positioned in a predetermined position on the shaft element and wherein the adjusting device is adapted to be coupeable to a retractor means.

## Description

### Surgical or medical retractor device

The present invention preferably relates to the field of surgical or medical devices. In particular, the present invention relates to a surgical retractor device to spread or pry apart an orifice of a body of a human or veterinary patient. In particular the present invention relates to a surgical retractor device to spread the chest and the thorax, respectively, or the abdomen of a human or veterinary patient.

During medical procedures in which for example an incision must be made between ribs of a ribcage of a patient and the upper portion of the ribcage has to be retracted or lifted to provide access to the viscera normally surgical retractors are used. Such surgical retractors can pry apart and keep open or lift an orifice of a body to allow a clear view or ready access.

In US 5,908,382 an intermammary harvesting retractor is disclosed. The retractor comprises an adjustable lifter blade assembly and an adjustable support tower assembly. The adjustable lifter blade assembly is comprised of a lifter blade receiver coupling fitting on flanges on the end of a movable arm of the adjustable support tower. The adjustable lifter blade receiver coupling comprises a clamping screw, a pair of hangers, and a center portion for receiving an adjustable lifter blade mounting flange. Further, the support tower assembly is comprised of a support bar mounted on a free end of a crossbar by a clamp and a support stand is mounted on the support bar by a clamp clamped by a thumbscrew. The cross bar of the support tower assembly which comprises a gear rack is connected to the movable arm by a crank mechanism to adjust the position of the movable arm relative to a fixed arm of the cross bar. The crank mechanism comprises a crank handle, wherein rotation of the crank handle moves the movable arm away from the fixed arm which remains locked in its adjusted position to retract the ribcage of the patient. A quick release locking mechanism locks the movable arm in an adjusted position. By closing handles of the quick release locking mechanism a gear of the locking mechanism is released from the gear rack allowing movable arm to be moved along the crossbar toward the fixed arm. When released the gear of the locking mechanism is then again engaged on the gear rack for adjustment by rotation of the crank handle.

The object of the invention is to provide an improved surgical or medical retractor device.

This object is solved by the retractor device with the features of claim 1.

According to the invention a retractor device is provided which comprises: a frame, a shaft element coupled to the frame, wherein the shaft element is provided with an adjusting device which is movably arranged on or along the shaft element to be positioned in a predetermined or defined position on the shaft element and wherein the adjusting device is adapted to be coupeable to a retractor means.

The adjusting device can be moved along the shaft element and positioned in a defined position on the shaft element, so that a distance between the adjusting device and a retractor means coupled to the adjusting device can be adjusted or adapted. Thus, the distance of a retractor means coupled to the adjusting device and the frame, which is fixed, e.g., to an operating table, can be varied in a wide range, without the need of further means such as, e.g., a winch.

Further embodiments and developments of the invention can be derived from the dependent claims and the description with reference to the figures.

In an embodiment of the invention the frame and the shaft element are coupled by a universal joint means. The universal joint allows a movement of the shaft element relative to the frame in all directions.

In another embodiment of the invention the universal joint means comprises a connecting means which is connectable to an arm device, wherein the arm device is connectable to the frame or is a part of the frame. Further, the connecting means is preferably movably along the arm device and more preferably movably along the arm device and fixable in a predefined position on the arm device.

In a further embodiment of the invention, the connecting means comprises two shoe elements which are positionable on opposite sides of the arm device and which are adapted to be movable relative to each other to adjust a gap in between, wherein the gap can be adjusted to be large enough to allow movement of the connecting means along the arm device and wherein the gap can be further adjusted to be small enough or zero to fix the connecting means on the arm device. The shoe element can be easily demounted and fumigated to be used, e.g., in an operating room.

In an embodiment of the invention, the universal joint means comprises a housing means in which a universal joint is housed. The housing means preferably comprises a base plate and a cover plate, wherein the base plate connects the housing means to the connecting means and the cover plate holds the universal joint means. Furthermore, the base plate and the cover plate are preferably releasably connected. The housing means can be also easily demounted and sufficiently disinfected or sanitized to be used for example in an operating room.

In another embodiment of the invention, the adjusting device comprises a connecting housing means which is movably arranged on or along the shaft element, wherein the connecting housing means is coupleable to the retractor means. The connecting housing means comprises a lever means which can be moved in a position in which the lever means is engaged with the shaft element and a position in which the lever means is disengaged from the shaft element. The lever means can be operated single-handed and therefore provides a comfortable actuation mechanism for a surgeon.

In a further embodiment of the invention, the lever means comprises an actuation section and an engagement section, wherein the lever means is arranged on the connecting housing means, so that when the actuation section is pushed, the engagement section is disengaged from the shaft element to allow movement of the connecting housing means and further, when the actuation section is released, the engagement section is engaged with the shaft element to fix the connecting housing means. The lever means can be actuated single-handed which facilitates handling of the adjusting device.

In an another embodiment of the invention the lever means comprises an actuation section and an engagement section, wherein the lever means is rotatably arranged on the connecting housing means, so that the actuation section can be rotated in a direction to disengage the engagement section and the shaft element to allow movement of the connecting housing means along the shaft element. Further, the actuation section can be rotated in the other direction to engage the engagement section and the shaft element to fix the connecting housing means. The lever means of the adjusting device can be easily positioned and fixed in a predetermined position on the shaft element by engaging the lever means with the shaft element in the intended or preferred position.

In an embodiment of the invention the shaft element comprises a plurality of recesses, wherein engagement section of the lever means comprises an engagement element which is adapted to be receivable in a recess of the shaft element. The actuation section can be actuated to move or to rotate the engagement section in a position in which the engagement element is received in a corresponding recess of the shaft element or removed from the corresponding recess of the shaft element. The recess is for example a slot, a through hole or a blind hole.

In a further embodiment of the invention the connecting housing means is adapted to be coupeable to the retractor means and/or coupeable to a connecting element, wherein the connecting element is adapted to be coupeable to the retractor means. Thus, connecting elements of difference lengths can be used to be connected with the connecting housing means to bridge different distances.

In another embodiment of the invention the connecting housing means is adapted to be releasably connected to the retractor means and/or the connecting element. Thus, the retractor means can be coupled or releasably connected to the connecting housing means in case, e.g., only a short distance has to be bridged and no additional connecting element is necessary to bridge the distance. Further, in case a distance between the adjusting device and a patient has to be bridged, which cannot be sufficiently bridged by connecting the connecting housing means and the retractor means, an additional connecting element can be used, having a suitable length to bridge the distance. This connecting element can then be coupled or connected to the connecting housing means and a retractor means can be coupled or connected to the connecting element.

In an embodiment of the invention the connecting element comprises a first rod element and a second rod element. The first rod element is adapted to be connectable to the connecting housing means. Further, the second rod element is adapted to be connectable at one end to the first rod element and at the other end to a receiving element and/or to the retractor means. Thus, the connecting element can be mounted and demounted, so that the connecting element and the connecting housing means can be easily disinfected or sanitized.

In a further embodiment of the invention the connecting housing means comprises a through hole through which the first rod element is passable. The first rod element comprises a first end and a second end, wherein the first end of the first rod element comprises a crank portion which is adapted to abut against the connecting housing means and wherein the second end of the first rod element is adapted to be fixable to the connecting housing means, e.g., by a nut. The second rod element is adapted to form an integral part with the first rod element or is adapted to be releasably connectable to the first rod element.

In another embodiment of the invention, the receiving element comprises a receiving means to receive a retractor means, wherein the receiving means is for example a ring or a hook. A ring or hook as a receiving means facilitates disinfection or sanitization. Further, a retractor means can be easily connected or coupled with the ring or hook of the receiving element.

A more detailed understanding of the invention may be had from the following description of preferred embodiments, given by way of example and to be understood in conjunction with the accompanying drawing, wherein:
- Fig. 1a: is a perspective view of a first embodiment of the retractor device according to the present invention;
- Fig. 1b: is a transparent view of the retractor device of Fig. 1a;
- Fig. 2: is a cross-sectional view of a part of the retractor device according to Figs. 1a and 1b;
- Fig. 3: is a perspective view of an example of a retractor means;
- Fig. 4: is a perspective view of a further example of a retractor means;
- Fig. 5: is a perspective view of another example of a retractor means;
- Fig. 6: is a perspective view of a further example of a retractor means;

- Fig. 7: is a cross sectional view of an example of a lever means and a corresponding shaft element of the retractor device of Figs. 1a and 1b;
- Fig. 8: is a cross sectional view of a further example of a lever means and a corresponding shaft element of the retractor device of Figs. 1a and 1b;
- Fig. 9: is a cross sectional view of another example of a lever means and a corresponding shaft element of the retractor device of Figs. 1a and 1b;
- Fig. 10: is a perspective view of a second embodiment of the retractor device according to the invention;
- Fig. 11: is a cross sectional view of an example of a lever means and a corresponding shaft element of the retractor device of Fig. 10; and
- Fig. 12: is a cross sectional view of the lever means and the corresponding shaft element of the retractor device of Fig. 11 in a release position.

In the figures the same reference numbers denote the same or functionally similar components, unless otherwise indicated. Further the illustrations in the figures are schematic and simplified and not drawn to scale.

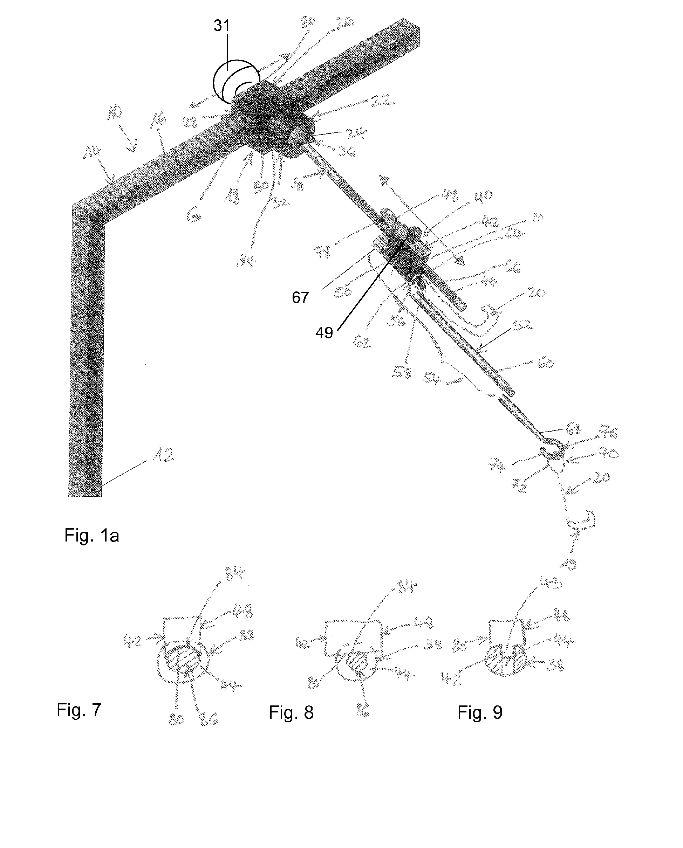

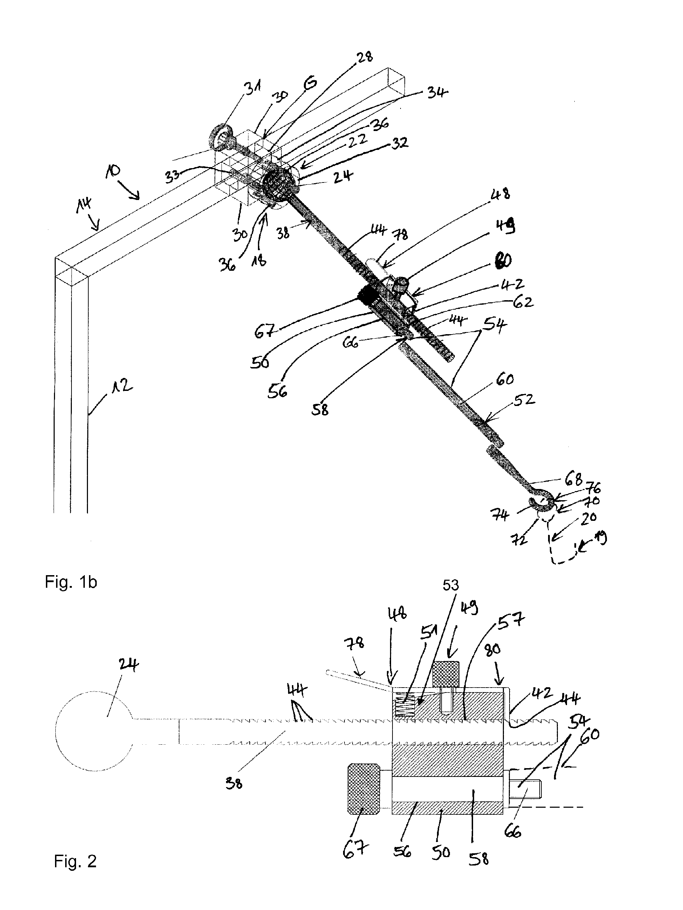

In Figs. 1a and 1b a perspective view of a first embodiment of a retractor device 10 according to the present invention is shown. In particular Fig. 1b shows a transparent view of the retractor device 10 of Fig. 1a. The retractor device 10 comprises a tower device or stand device 12 partially shown in Figs. 1a and 1b which is designed to be fixed to a base means (not shown), e.g., an operating table. The retractor device 10 can be, e.g., releasably fixed to a base means such as an operating means by a fixation means such as, e.g., a clamping device to clamp the retractor device 10 on the base means. Such fixation means to fix a retractor device to a base means such as an operating table are commonly known.

Further, an arm device 14 is connected to the stand device 12. The stand device 12 and the arm device 14 can be either separately or releasably connected (not shown) or integrally connected as shown in Figs. 1a and 1b, in which the arm device 14 and the stand device 12 form a single part. The arm device 14 comprises a shaft 16, e.g. a duct, a tube or a rod, wherein the shaft 16 can be provided, e.g., with a squared or rectangular cross-section as shown in Figs. 1a, 1b. However, the shaft 16 can have any cross-section, e.g., a rectangular, circular and/or oval cross-section etc..

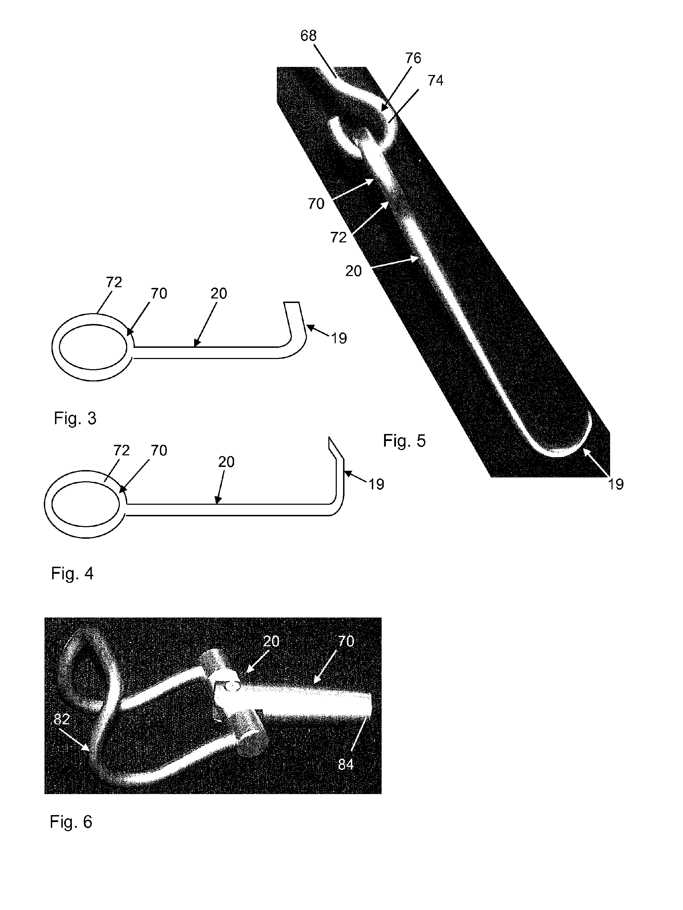

Further, a universal joint means 18 or peripheral joint means is provided on the arm device 14 which can be connected or coupled to a retractor means 20, e.g. a blade device or a hook device 19 as shown in Fig. 1a etc., to retract, spread, lift or pry apart an opening in a body of a human or veterinary patient, e.g., an opened ribcage, thorax or abdomen etc.. Some examples for retractor means 20 will be shown in Figs. 3 to 6 below. The universal joint means 18 is either fixedly (not shown) or movably mounted on the arm device 14 to position the universal joint means 18 in a defined distance or defined position relative to the opening of the patient to be retracted. As indicated by the arrow in Figs. 1a, 1b the universal joint means 18 in the embodiment as shown can be moved or slided along the arm device 14 forward and backward or towards and away from the opening of the body to be retracted. Further, the universal joint means 18 is preferably designed to be fixed at said defined or intended position on the arm device 14.

As shown in Figs. 1a and 1b the universal joint means 18 comprises a housing means 22, wherein the housing means 22 houses a universal joint 24 or peripheral joint. Further, the universal joint means 18 comprises a connecting means 26 to connect the housing means 22 and the universal joint 24 to the arm device 14. The connecting means 26 is preferably designed as mentioned above, so that the universal joint means 18 can be moved or slided along the arm device 14 as shown by the arrow and, more preferably, can be additionally fixed in a defined position on the arm device 14 and its shaft 16, respectively.

In the example shown in Figs. 1a, 1b the connecting means 26 comprises an opening 28 through which the arm device 14 and its shaft 16, respectively, can be passed. The opening 28 of the connecting means 26 is formed for example by two shoe elements 30 which are arranged on opposite sides of the arm device 14 and its shaft 16 to form the opening 28 and further an adjustable gap G in between. As shown in Figs. 1a, 1b the shoe elements 30 have, e.g., a U-shape which corresponds to the rectangular cross-section of the shaft 16 of the arm device 14.

The gap G between the ends of the shoe elements 30 is preferably adjustable so that the connecting means 26 and its shoe elements 30 can be moved along the arm device 14 and fixed in a certain position. In other words, the gap G formed by the shoe elements 30 can be adjusted so that the gap G is large enough to allow that the universal joint means 18 and its connecting means 26 can be moved along the arm device 14 and further the gap G can be adjusted so that the gap G is small enough or zero so that the universal joint means 18 and its connecting means 26 can be fixed or clamped in a defined or preferred position on the arm device 14.

To adjust the gap G between the shoe elements 30, e.g., a screw means 31 such as for example at least one locking screw or fixing screw, shown in Figs. 1a and 1b can be used to move the shoe elements 30 relative to each other, so that the shoe elements 30 can be screwed apart from each other or together to adjust the gap G in between. As shown in the transparent view of Fig. 1b an additional pin 33 or bolt can be arranged between the two shoe elements 30 to hold them in position, when the shoe elements 30 are moved relative to each other by actuating the locking screw or fixing screw 31 in Fig. 1b.

However, the invention is not restricted to this particular embodiment of a connecting means 26 for a universal joint 24 or peripheral joint which is movable along the arm device 14 and preferably fixable in a defined position thereon. Any other connecting means can be also used which is suitable to connect a universal joint 24 or peripheral joint to an arm device 14, wherein the universal joint or peripheral joint 24 can be moved along the arm device 14 and its shaft 16, respectively, and preferably fixed in a certain position thereon.

As shown in Figs. 1a, 1b, the housing means 22 of the universal joint means 18 is preferably designed to be demountable to facilitate, e.g., disinfection of the housing means 22 and the universal joint 24. As can be derived from Figs. 1a, 1b the housing means 22 comprises a cover plate 32 which is connected to a base plate 34 by screws 36. The universal joint 24 is movably or rotatably located between the base plate 34 and the cover plate 32, wherein the cover plate 32 is designed to hold the universal joint 24, so that the universal joint 24 cannot be unintentionally detached from the housing means 22. The base plate 34 can form an integral part of the connecting means 26, as shown in Fig. 1, or a separate part which is fixed at the connecting means 26, e.g., by the screws 36 etc..

Further, the universal joint 24 of the universal joint means 18 comprises a shaft element 38, wherein the shaft element 38 can form a separate part which is releasably connected to the universal joint 24, e.g., by screwing etc., so that different shaft elements 38 can be connected with the universal joint 24, for example shaft elements having a different length etc.. In an alternative embodiment of the inventive retractor device the shaft element can also form an integral part or single part with the universal joint (not shown).

As shown in Figs. 1a, 1b the retractor device 10 is designed so that the distance of the retractor means 20, e.g., a hook device 19 or a blade device as shown in Fig. 1b and further below in Fig. 6, can be adjusted or operated single-handed by a user such as a surgeon.

According to the first embodiment of the inventive retractor device 10, as shown in Figs. 1a, 1b, the retractor device 10 is designed to be operated or adjusted by an adjusting device 40. The adjusting device 40 comprises a lever means 48 which is provided with an engagement element 42 which can be brought in engagement with the shaft element 38. As shown in Figs. 1a, 1b, the shaft element 38 therefore comprises at least one or a plurality of recesses 44, e.g. in the form of slots 46 or bores or through-holes (not shown), to receive and fix or hold the engagement element 42 of the adjusting device 40. According to the invention the lever means 48 can be easily operated by only one hand to adjust the adjusting device 40 at a preferred position or distance on the shaft element 38.

The adjusting device 40 comprises a connecting housing means 50 which is movably located on the shaft element 38 and which further connects the shaft element 38 and the retractor means 20, e.g. a hook device 19, as shown in Fig. 1a. As can be derived from Figs. 1a, 1b, the connecting housing means 50 comprises a receiving section 52 for receiving the retractor means 20, e.g., a hook device 19 or a blade device etc..

In principle the retractor means 20 can be directly connected to the connecting housing means 50 as indicated by the dashed-dotted lines in Fig. 1a, e.g., by forming an integral part with the connecting housing means 50 or by releasably connecting the retractor means 20 and the connecting housing means 50, .e.g., by screwing etc..

In the example shown in Figs. 1a, 1b the retractor means 20 is connected to the connecting housing means 50 via a connecting element 54. The additional connecting element 54 has the advantage that larger distances between the retractor device 10 and an opening of a patient to be retracted can be bridged. Otherwise a sufficiently long retractor means 20 can be also used.

As shown in the transparent view of the retractor device 10 in Fig. 1b and further in the cross-sectional view of the universal joint and the adjusting device in Fig. 2 the connecting housing means 50 comprises for example a passage or a through-hole 56 through which the connecting element 54 can be passed. To secure the connecting element 54, preferably releasably, to the connecting housing means 50, the connecting element 54 comprises for example a first rod element 58 and a second rod element 60. The first rod element 58 is passed through the through-hole 56 of the connecting housing means 50, wherein the first rod element 58 comprises, e.g., a crank portion 62, which abuts against the connecting housing means 50 when the first rod element 58 is passed through the through-hole 56. Further, the first rod element 58 comprises a connecting portion 64, e.g., a thread 66, to be connected to the second rod element 60. Further, the first rod element 58 is secured to the connecting housing means 50 for example by a nut element 67 as shown in Figs. 1a, 1b and 2.

The second rod element 60 is further designed to be connected to the retractor means 20. The second rod element 60 as shown in Figs. 1a, 1b, can be releasably connected to a receiving element 68 which receives and holds the retractor means 20, such as for example a hook device 19 (see Fig. 1a). In an alternative embodiment the second rod element 60 can also form a single part with the receiving element 64 which is connectable to the retractor means 20 (not shown).

As shown in Figs. 1a, 1b the retractor means 20 comprises a coupling element 70 to be coupled or connected with the receiving element 68 of the connecting element 54 and its second rod element 60, respectively. In the example shown in Fig. 1a the coupling element 70 comprises for example a ring element 72 at one end which can be received by the receiving element 68 of the connecting element 54. The receiving element 68 of the connecting element 54 and the second rod element 60, respectively, comprises for example a hook or hook portion 74 to hook in or into the ring element 72 of the retractor means 20.

The releasable connection of the second rod element 60 and the receiving element 68 has the advantage that different second rod elements 60, having for example different lengths, can be combined with the receiving element 68. Further, different receiving elements 68 which can be connected or coupled with different retractor means 20 can be combined with the second rod element 60. That means, that receiving elements 68 which comprise as a receiving means 76 for example a hook 74, or a ring element (such as the retractor means 20 in Fig. 1a) or any other form of receiving means 76 can be combined with corresponding coupling elements 70 of retractor means 20. Such a coupling element 70 can comprise for example a ring element 72 as shown in Fig. 1a or a hook similar to the hook 74 of the receiving element 68 in Figs. 1a, 1b or any other element which is suitable to be connected to a corresponding receiving means 76 of a receiving element 68.

However, the invention is not limited to the particular example of the receiving element 68, its receiving means 76 and the retractor means 20 and its coupling element 70 shown in Figs. 1a, 1b and following Figs. 3 to 6. The design of the receiving element 68 and its receiving means 76 depends on the connection or coupling to the retractor means and its coupling element 70 and vice versa. In case the retractor means 20 comprises as a coupling element 70, e.g., a hook, to be connected to the receiving means 76 of the connecting element 54 (not shown), the receiving means 76 can be also designed, e.g., as a ring element in which the hook of the retractor means 20 can be hooked in or into. It is further obvious for the person skilled in the art, that any other connecting element 54 and retractor means 20 can be used which are suitable to be connected with each other.

In case now the retractor means 20 is inserted in an opening of a body of a patient, e.g., an opened ribcage, the retractor means 20 can be moved by using only one hand in a retracted or in a relaxed position by actuating the adjusting device 40, as shown in Figs. 1a, 1b and 2.

When moving the adjusting device 40 and its connecting housing means 50 in Figs. 1a, 1b and 2 forward and backward along the shaft element 38 the retractor means 20 can be moved towards and away from a patient, so that when the retractor means 20 is inserted in an opening of the patients body, the opening can be retracted or relaxed. The lever means 48 is attached to or fixed on the connecting housing means 50, e.g., by a screw 49 or screw element. To move the connecting housing means 50 the lever means 48 is actuated by depressing one end of the lever means 48 which forms the actuation section 78 so that the other end of the lever means 48 which forms the engagement section 80 and which comprises the engagement element 42 is disengaged or released from the shaft element 38. That means the engagement section 80 and its engagement element 42 of the lever means 48 is removed from its corresponding recess 44 in the shaft element 38. Thus, the connecting housing means 50 and the retractor means 20 coupled to the connecting housing means 50 via the connecting element 54 can be moved or slided along the shaft element 38 to for example sufficiently retract of pry apart the opening in the body of the patient.

To fix the connecting housing means 50 and thus the retractor means 20 in an intended position, the lever means 48 can be released, so that the engagement element 42 of the lever means 48 is received in a corresponding recess 44 of the shaft element 38, so that the adjusting device 40 cannot be moved unintentionally.

Preferably the lever means 48 and the connecting housing means 50 are releasably connected, so that they can be demounted, e.g., for disinfection. As shown in Figs. 1a, 1b and 2 the lever means 48 is fixed to the connecting housing means 50, e.g., by a screw 49. Furthermore, the lever means 48 comprises in the example shown on Figs. 1a, 1b and 2 an elongated end forming the actuating section 78, wherein said elongated end or actuation section 78 is either straight or inclined, preferably inclined upward, so that when the actuation section 78 is pressed downward the engagement section 80 and its engagement element 42 can be released from the shaft element 38 and its corresponding recess 44, so that the connecting housing means 50 can be moved along the shaft element 38.

As described before, the shaft element 38 which is connected to the universal joint 24 comprises a plurality of recesses 44 or slots, e.g. circumferential slots, which are spaced apart from each other by a predefined distance, e.g., a distance in a range between 1mm to 2mm. However any other distance between the recesses 44 which is smaller than 1mm or larger than 2mm can be chosen as well. Further, the recesses 44 can be spaced apart from each other by the same distance or by different distances.

In Fig. 2 a cross-sectional view is shown of a part of the retractor device 10 of Figs. 1a and 1b. As shown in Fig. 2 the universal joint 24 is releasably connected to the shaft element 38, wherein the universal joint 24 and the shaft element 38 are screwed together. Further, the connecting housing means 50 of the adjusting device 40 is movably arranged in a through-hole 57 of the shaft element 38. As shown in Fig. 2 the connecting housing means 50 comprises a through-hole in which the shaft element 38 is movably or slidably arranged. The connecting housing means 50 further comprises the lever means 48, wherein the actuation section 78 of the lever means 48 is for example inclined. As shown in Fig. 2, the lever means 48 can be attached to the connecting housing means 50 by a screw 49 to fix the lever means 48, so that the actuation section 78 of the lever means 48 can be pushed down to lift up the engagement section 80 of the lever means 48 and disengage the engagement element 42 from the shaft element 38 and its corresponding recess 44.

Furthermore, an additional spring or spring element 51 can be provided between the connecting housing means 50 and the lever means 48 and its actuation section 78 which pretensions the lever means 48 and its actuation section 78, so that the engagement section 80 of the lever means 48 is additionally pressed in engagement with the shaft element 38 and a corresponding recess 44 of the shaft element 38, as shown in Fig. 2. On the other hand, when the lever means 48 and its actuation section 78 is pressed down the spring element 51 is compressed and further the engagement section 80 of the lever means 48 is lifted and brought out of engagement with the corresponding recess 44 of the shaft element 38, so that the adjusting device 40 and a retractor means coupled with the adjusting device 40 can be moved in a preferred or intended position to, e.g., further pry apart an opening in the body of a human or veterinary body. Furthermore, as can be derived from Fig. 2, a portion 53 of the connecting housing means 50 can be inclined to facilitate pushing down of the lever means 48.

Fig. 2 also shows, as described before, the other passage or through-hole 56 of the connecting housing means 50 through which the connecting element 54 is passed. As shown in Fig. 2, the first rod element 58 is shown, which is passed through the through-hole 56, wherein the first rod element 58 comprises for example a crank portion 62, which abuts against the connecting housing means 50 when the first rod element 58 is passed through the through-hole 56. Furthermore, the first rod element 58 comprises a connecting portion 64, e.g., a thread 66, to be connected to the second rod element 60, which is indicated by the dashed lines in Fig. 2, or to a receiving element 68 (not shown). As shown in Fig. 2, the first rod element 58 is secured to the connecting housing means 50 for example by a nut element 67.

In Figs. 3, 4, 5 and 6 several examples for retractor means 20 are shown. The retractor means in Figs. 3 and 4 are schematic. As can be derived from Figs. 3, 4 and 5 the hook devices 19 are of different size. The hook device 19 in Fig. 5 is the largest and the hook device 19 in Fig. 3 is the smallest of all three hook devices 19. The three hook devices 19, as shown as examples of a retractor means 20 in Figs. 3, 4 and 5, comprise, e.g., hooks of different size and further comprise different lengths. Further all hook devices 19 comprise a ring element 72 as a coupling element 70, so that a receiving means 76 in the form of a hook 74, as shown in Fig. 5, can be hooked in the ring element 72 to connect or couple the retractor means 20 to the receiving element 68 of a retractor device 10 according to the invention.

Further, Fig. 6 shows another example of a retractor means 20. In the present case the retractor means 20 comprises a blade element 82. The blade element 82 comprises as a coupling element 70, e.g., a hollow shaft 84 which is provided with an internal thread. Thus, the blade element 82 can be screwed, e.g., on the end of a second rod element, which is provided with a corresponding external thread. In this case no additional receiving element 68, as shown above in Fig. 5, is necessary to couple or connect the connecting element and the retractor means 20. It is obvious to a person skilled in the art, that the invention is not restricted to the examples of retractor means 20 and the way they are connected or coupled to the connecting housing means.

In Fig. 7 a cross sectional view of an example of a lever means 48 and a corresponding shaft element 38 of the retractor device 10 of Figs. 1a, 1b and 2 is shown.

As can be derived from Fig. 7 the engagement section 80 and the engagement element 42 of the lever means 48 can be adapted according to the form or cross-section 86 of the corresponding recess 44. In the present case, the engagement section 80 and its engagement element 42 can be provided with a rounded end 84 as shown in Fig. 7 corresponding to the rounded shape cross-section 86 of the recess 44.

In Fig. 8 a cross sectional view of another example of a lever means 48 and a corresponding shaft element 38 of the retractor device 10 of Figs. 1a, 1b and 2 is shown.

As can be derived from Figs. 8 the engagement section 80 and its engagement element 42 of the lever means 48 must not have to be adapted according to the form or cross-section 86 of the recess 44. Therefore the engagement section 80 of the lever means 48 and its engagement element 42 can be also provided, e.g., with a straight end 84 while the corresponding recess 44 has a circular cross-section 86.

In Fig. 9 a further embodiment of a lever means 48 and a shaft element 38 and its recesses 44 is shown. Fig. 9 shows a cross sectional view of a further example of a lever means 48 and a corresponding shaft element 38 of the retractor device 10 of Figs. 1a, 1b and 2. In Fig. 9 the recesses 44 are formed as holes, e.g., through-holes as shown in Fig. 9. The engagement element 42 of the lever means 48 comprises a corresponding protrusion 43 which can be received in the hole 44 of the shaft element 38, so that the adjustable device can be fixed in a defined position on the shaft element 38. Further, the lever means 48 and is actuation section can be pushed down to lift up and remove the engagement element 42 and its protrusion 43, respectively, from the recess 44 or hole of the shaft element 38, so that the adjustable device can be moved along the shaft element 38 to retract or to relax a retractor means coupled to the retractor device.

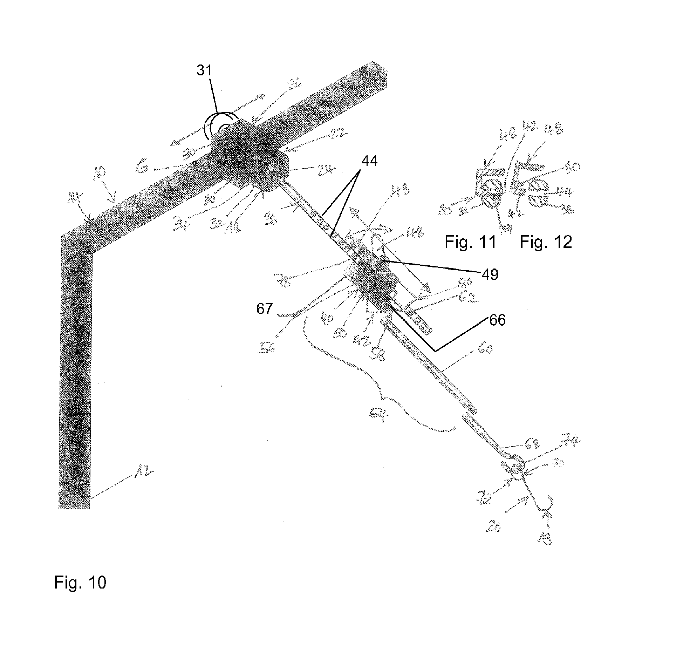

Fig. 10 shows a partial view of a second embodiment of the retractor device 10 according to the invention. The retractor device 10 of the second embodiment differs from the retractor device 10 of the first embodiment, as described above, e.g., with respect to Figs. 1a, 1b and 2, in that the retractor device 10 of the second embodiment comprises a lever means 48 which is rotatebly, so that the lever means 48 can be rotated into an engagement position (see following Fig. 11) and further, as shown by the dashed lines in Fig. 10, out off the engagement position with the shaft element 38, namely in a release position or disconnected position from the shaft element 38 and a corresponding recess 44 (see following Fig. 12). Therefore, the elements which the first and the second embodiment have in common will be only briefly described with respect to Fig. 10. In this connection reference is made to the description of Figs. 1a, 1b and 2 above in which all elements of the first embodiment are described in detail.

As can be derived from Fig. 10, the retractor device 10 of the second embodiment comprises same as the retractor device 10 of the first embodiment, as described before with respect to Figs. 1a, 1b, a tower device or stand device 12, which is designed to be preferably releasably fixed to a base means (not shown), e.g., an operating table etc.. Further, the retractor device 10 comprises an arm device 14 which is connected to the stand device 12. Furthermore, a universal joint means 18 or peripheral joint means is provided on the arm device 14 and can be connected or coupled to a retractor means 20, e.g., a blade device or a hook device 19 etc., to, e.g., retract, spread, lift or pry apart an opening in a body of a human or veterinary patient.

As shown in Fig. 10 the universal joint means 18 comprises a housing means 22, wherein the housing means 22 houses a universal joint 24 or peripheral joint and comprises a cover plate 32 and a base plate 34. Further, the universal joint means 18 comprises a connecting means 26 to connect the housing means 22 and the universal joint 24 to the arm device 14. The connecting means 26 comprises, e.g., two shoe elements 30 which are arranged on opposite sides of the arm device 14 to form an opening 28 so that the arm device 14 can be passed through. Further, the two shoe elements 30 form an adjustable gap G in between. To adjust the gap G between the shoe elements 30, e.g., a screw means 31 (e.g., a fixation screw or set screw) can be used to move the shoe elements 30 relative to each other, so that the shoe elements 30 can be screwed apart from each other or together to adjust the gap G in between. In this connection an additional pin 33 or bolt can be arranged between the two shoe elements 30 to hold them in position, when the shoe elements 30 are moved relative to each other by actuating the locking screw or fixing screw 31.

Further, the universal joint 24 of the universal joint means 18 is connected to a shaft element 38, wherein the shaft element 38 can form a separate part which is releasably connected to the universal joint 24, e.g., by screwing etc. as shown in Fig. 10, or an integral part or single part with the universal joint (not shown).

According to the invention, the retractor device 10 is designed so that the distance of the retractor means 20, e.g., a blade device or hook device 19 etc., can be adjusted or operated preferably single-handed by a user such as a surgeon.

Therefore the second embodiment of the inventive retractor device 10, as shown in Fig. 10, is designed to be operated or adjusted by an adjusting device 40. The adjusting device 40 comprises a lever means 48, wherein the lever means 48 comprises an engagement section 80 with an engagement element 42 which can be brought in engagement with the shaft element 38. As shown in Fig. 10 the shaft element 38 therefore comprises at least one or a plurality of recesses 44, e.g., in the form of slots (see Figs. 1a, 1b, 2) or holes (e.g. through-holes or through bores, see Fig. 10), to receive and fix or hold the engagement element 42.

According to the second embodiment the lever means 48 is designed as a rotatable lever means 48 which can be easily operated by only one hand to adjust the adjusting device 40 at a preferred position or distance on the shaft element 38.

The adjusting device 40 comprises a connecting housing means 50 which is movably located on the shaft element 38, as described before, e.g., with respect to Figs. 1a, 1b and 2. The adjusting device 40 connects the shaft element 38 and the retractor means 20, e.g., a hook device 19, as shown in Fig. 10, either directly or by using an additional receiving element 68 (see Fig. 10). In principle the retractor means 20 can be directly connected to the connecting housing means 50 as shown exemplary in Fig. 1a before, e.g., by forming an integral part with the connecting housing means 50 or by releasably connecting the retractor means 20 and the connecting housing means 50 as shown in Fig. 10, by using, e.g., a connecting element 54 comprising a first rod element 58 and a second rod element 60. The second rod element 60 can be, e.g., releasably connected to the receiving element 64 by screwing both parts together. The receiving element 64 comprises, e.g., a hook 74 in which a ring element 72 of a hook device 19 (retractor means 20) can be hooked in or into, as shown in Fig. 10.

As shown in Fig. 10 the connecting housing means 50 comprises for example a passage or a through-hole 56 through which the first rod element 58 of the connecting element 54 can be passed and fixed to the connecting housing means 50. To secure the first rod element 58 to the connecting housing means 50, the first rod element 58 comprises, e.g., a crank portion 62 which abuts against the connecting housing means 50, when passed through the through-hole 56. On the other side of the through-hole 56, the first rod element 58 is fixed, e.g., by a nut 67. The first rod element 58 and the second rod element 60 of the connecting element 54 can be further connected to the receiving element 68 to receive and fix or hold the retractor means 20. As described before with respect to Figs. 1a, 1b and 2 in further embodiments of the inventive retractor device 10, the receiving element 68 can be for example either omitted or can form a single part or integral part with the connecting element 54 or the connecting housing means 50. In case the receiving element 68 is connected to the connecting element 54, the receiving element 68 can be connected to the second rod element 60 as shown in Fig. 10, or can be connected to the first rod element 58 by screwing the receiving element 68 on the first rod element 58. In this case, the second rod element 60 of the connecting element 54 is omitted. This applies to all embodiments of the invention.

As shown and as described before, e.g., with respect to Fig. 2, the shaft element 38 in Fig. 10 can be movably or slidably located in a through hole of the connecting housing means 50.

Further, as shown in Fig. 10 the retractor means 20 comprises a coupling element 70 to be coupled with the receiving element 68. In the example shown in Fig. 10 the coupling element 70 comprises for example a ring element 72 at one end which receives the receiving element 68. The receiving element 68 comprises for example a hook 74 to hook in or into the ring element 72 of the retractor means 20 as described before.

In case now the retractor means 20 is inserted in an opening of a body of a patient, e.g., an opened ribcage, the retractor means 20 can be moved in a retracted or in a relaxed position by actuating the adjusting device 40 and rotating the lever means 48, so that its engagement section 80 and its engagement element 42 is not received in a recess 44 of the shaft element 38, as indicated by the dashed line in Fig. 10.

When moving the adjusting device 40 and its connecting housing means 50 forward and backward along the shaft element 38 the retractor means 20 can be moved torwards and away from a patient, so that when the retractor means 20 is inserted in an opening of the patient's body, the opening can be retracted or relaxed. To move the connecting housing means 50 the lever means 48 is actuated by rotating the lever means 48 as mentioned before. The lever means 48 is rotatebly fixed to the connecting housing means 50, e.g., by a screw 49, wherein the screw 49 is attached to the connecting housing means 50, so that the lever means 48 is fixed on one hand on the connecting housing means 50 but still can be sufficiently rotated. An additional spring as shown in Fig. 2 above is not necessary.

The lever means 48 comprises an actuation section 78 to rotate or turn the lever means 48 around the screw 49 and thus to engage or disengage the engagement section 80 and its engagement element 42 from a corresponding recess 44 of the shaft element 38, e.g. a hole as shown in Fig. 10 or a slot etc..

To retract or pry apart an opening of a body of a patient, the lever means 48 is rotated or turned so that its engagement section 80 and its engagement element 42 is out of contact with the shaft element 38 and its corresponding recess 44, respectively. This means that the connecting housing means 50 and the retractor means 20 coupled to the connecting housing means 50 (via the connecting element 54) can be moved or slided along the shaft element 38 for example to sufficiently retract of pry apart the opening in the body of the patient. To fix the connecting housing means 50 and thus the retractor means 20 in an intended position, the lever means 48 is rotated in an engagement position in which its engagement section 80 and its engagement element 42 (i.e. a protrusion as shown in Fig. 10) is received in a corresponding recess 44 or through-hole of the shaft element 38 as shown in Fig. 10. In this engagement position the lever means 48 and its engagement section 80 is fixed by the shaft element 38, so that the adjusting device 40 cannot be moved unintentionally. Further, the adjusting device 40 can be fixed in a defined distance to the opening of the body of the patient. Instead of a recess 44 in the form of a hole, e.g. a through hole or a blind hole, also recesses 44 in the form of slots as shown before in Figs. 1a, 1b and 2 can be used and can be provided on the shaft element 38 to receive an engagement section 80 of the lever means 48. The engagement section 80 can comprise a corresponding protrusion, e.g., a tip end or pin at the end as an engagement element which can be received in a corresponding recess 44 of the shaft element 38, wherein the recess 44 is for example a hole or a bore.

In Fig. 11 a cross section of the shaft element 38 and the lever means 48 and its engagement section 80 and engagement element 42 is shown. In Fig. 11 the lever means 48 is in an engagement position in which the engagement element 42, i.e. a protrusion, is received in a corresponding recess 44 or hole of the shaft element 38, so that the connecting housing means 50 is fixed and cannot be moved along the shaft element.

Further, in Fig. 12 a cross section is shown of the shaft element 38, the lever means 48, its engagement section 80 and engagement element 42 of Fig. 11. In Fig. 12 the lever means 48 is in an release or disengaged position, in which the engagement element 42, i.e. a protrusion, is removed from the corresponding recess 44 or hole of the shaft element 38, so that the connecting housing means 50 can be moved along the shaft element 38 to retract or to relax a retractor means connected with the retractor device 10.

While this invention has been particularly shown and described with reference to preferred embodiments thereof, it will be understood by those skilled in the art that various changes in form, modification, variation and details may be made therein without departing from the scope of the invention as defined by the appended claims. Further, the embodiments as described before in Figs. 1 to 12 can be also combined, at least single features of the embodiments.

List of reference signs
- 10: retractor device
- 12: stand device
- 14: arm device
- 16: shaft (of arm device)
- 18: universal joint means
- 19: hook device
- 20: retractor means
- 22: housing means (universal joint means)
- 24: universal joint
- 26: connecting means (universal joint means)
- 28: opening (connecting means)
- 30: shoe elements
- 31: fixing screw
- 32: cover plate (housing means of universal joint means)
- 33: pin (shoe element)
- 34: base plate (housing means of universal joint means)
- 36: screw
- 38: shaft element
- 40: adjusting device
- 42: engaging element
- 43: protrusion
- 44: recess
- 46: slot
- 48: lever means
- 49: screw (lever means)
- 50: connecting housing means
- 51: spring (lever means)
- 52: receiving section (of connecting housing means)
- 53: portion (of connecting housing means)
- 54: connecting element
- 56: through-hole (for first rod element 58)
- 57: through-hole (for shaft element 38)

- 58: first rod element
- 60: second rod element
- 62: crank portion
- 64: connecting portion
- 66: thread
- 67: nut (first rod element)
- 68: receiving element
- 70: coupling element (retractor means)
- 72: ring element (coupling element)
- 74: hook (receiving element)
- 76: receiving means
- 78: actuation section (lever means)
- 80: engagement section (lever means)
- 82: blade device
- 84: end of engagement section (engagement element)
- 86: cross-section of recess
- 88: protrusion (engagement section)
- 90: through hole (recess)

## Claims

1. Surgical retractor device comprising:
a frame (12), a shaft element (38) coupled to the frame (12),
wherein the shaft element (38) is provided with an adjusting device (40) which is movably arranged along the shaft element (38) to be positioned in a predetermined position on the shaft element (38) and wherein the adjusting device (40) is adapted to be coupeable to a retractor means (20).

2. Surgical retractor device according to claim 1,
**wherein**,
the frame (12) and the shaft element (38) are coupled by a universal joint means (18).

3. Surgical retractor device according to claim 2,
**wherein**,
the universal joint means (18) comprises a connecting means (26) which is connectable to an arm device (14), wherein the arm device (14) is connectable to or part of the frame (12) and wherein the connecting means (26) is preferably movably along the arm device (14) and more preferably movably along the arm device (14) and fixable in a predefined position on the arm device (14).

4. Surgical retractor device according to claim 3,
**wherein**,
the connecting means (26) comprises two shoe elements (30) which are positionable on opposite sides of the arm device (14) and which are adapted to be movable relative to each other to adjust a gap (G) in between, wherein the gap (G) can be adjusted to be large enough to allow movement of the connecting means (26) along the arm device (20) and wherein the gap (G) can be further adjusted to be small enough or zero to fix the connecting means (20) on the arm device.

5. Surgical retractor device according to any of claims 2 to 4,
**wherein,**
the universal joint means (18) comprises a housing means (22) in which a universal joint (24) is housed and wherein the housing means (22) preferably comprises a base plate (34) and a cover plate (32), wherein the base plate (34) connects the housing means (22) to the connecting means (26) and the cover plate (32) holds the universal joint means (18), and wherein the base plate (34) and the cover plate (32) are preferably releasably connected.

6. Surgical retractor device according to any of claims 1 to 5,
**wherein,**
the adjusting device (40) comprises a connecting housing means (50) which is movably arranged along the shaft element (38), wherein the connecting housing means (50) is coupleable to the retractor means (20), and wherein the connecting housing means (50) comprises a lever means (48) which can be moved in a position in which the lever means (48) is engaged with the shaft element (38) and a position in which the lever means (48) is disengaged from the shaft element (38).

7. Surgical retractor device according to claim 6,
**wherein**,
the lever means (48) comprises an actuation section (78) and an engagement section (80), wherein the lever means (48) is arranged on the connecting housing means (50) so that when the actuation section (78) is pushed, the engagement section (80) is disengaged from the shaft element to allow movement of the connecting housing means (50) and further, when the actuation section (78) is released the engagement section (80) is engaged with the shaft element (38) to fix the connecting housing means (50).

8. Surgical retractor device according to claim 6 or 7,
**wherein** ,
the lever means (48) comprises an actuation section (78) and an engagement section (80), wherein the lever means (48) is rotatably arranged on the connecting housing means (50) so that the actuation section (78) can be rotated in a direction to disengage the engagement section (80) and the shaft element (38) to allow movement of the connecting housing means (50) and further, the actuation section (78) can be rotated in the other direction to engage the engagement section (80) and the shaft element (38) to fix the connecting housing means (50).

9. Surgical retractor device according to any of claims 7 or 8,
**wherein,**
the shaft element (38) comprises a plurality of recesses (44), wherein engagement section (80) of the lever means (48) comprises an engagement element (42) which is adapted to be receivable in a recess (44) of the shaft element (38), wherein the actuation section (80) can be actuated to move or rotate the engagement section (80) in a position in which the engagement element (42) is received in a corresponding recess (44) of the shaft element (38) or removed from the corresponding recess (44) of the shaft element (38), and wherein the recess (44) is in particular a slot, a through hole or a blind hole.

10. Surgical retractor device according to any of claims 6 to 9,
**wherein,**
the connecting housing means (50) is adapted to be coupeable to the retractor means (20) and/or coupeable to a connecting element (54), wherein the connecting element (54) is adapted to be coupeable to the retractor means (20).

11. Surgical retractor device according to claim 10,
**wherein** ,
the connecting housing means (50) is adapted to be releasably connected to the retractor means (20) and/or the connecting element (54).

12. Surgical retractor device according to any of claims 10 or 11,
**wherein,**
the connecting element (54) comprises a first rod element (58) and a second rod element (60), wherein the first rod element (58) is adapted to be connectable to the connecting housing means (50) and the second rod element (60) is adapted to be connectable at one end to the first rod element (58) and at the other end to a receiving element (68) or to the retractor means (20).

13. Surgical retractor device according to claim 12,
**wherein**,
the connecting housing means (50) comprises a through hole (56) through which the first rod element (58) is passable, wherein the first rod element (58) comprises a first and second end, wherein the first end of the first rod element comprises a crank portion (62) which is adapted to abut against the connecting housing means (50) and wherein the second end of the first rod element (58) is adapted to be fixable to the connecting housing means (50), in particular by a nut (67), and wherein the second rod element (60) is adapted to form an integral part with the first rod element (58) or is adapted to be releasably connectable to the first rod element (58).

14. Surgical retractor device according to claims 12 or 13,
**wherein**,
that receiving element (68) comprises a receiving means (76) to receive a retractor means (20), wherein the receiving means (76) is in particular a ring or a hook (74).
